# EUROPEAN PATENT APPLICATION

(11) **EP 2 036 570 A1**
(43) Date of publication of application: **18.03.2009**
(21) Application number: 07116335.6
(22) Date of filing: 13.09.2007
(51) Int. Cl.: A61K 38/17, A61L 33/00, A61P 9/00

(54) **Means and methods for influencing platelet-collagen interaction**

(71) Applicant: UMC Utrecht Holding B.V., 3584 CM Utrecht (NL)
(72) Inventor: The designation of the inventor has not yet been filed
(74) Representative: Hatzmann, Martin

(57) **Abstract**

The invention provides means and methods for influencing platelet-collagen interaction using LAIR, or a functional equivalent thereof, and/or a compound capable of specifically interacting in the binding of LAIR and collagen.

## Description

The invention relates to the fields of biology, medicine and hematology.

The formation of platelet-rich thrombi is a multistep process involving several components (for review see Denis CV and Wagner DD (2007) ATVB 27:728). The initial step of thrombus formation requires the capturing of platelets from flowing blood to the exposed subendothelial matrix of an injured vessel wall. Once bound to this matrix, platelets become activated, allowing the formation of platelet-platelet interactions which is needed for thrombus growth. Platelet activation is enhanced via thrombin, a product of the coagulation cascade or via components (such as ADP, epinephrine and thromboxane A2) that are released from platelets. Finally, this primary activation and aggregation step is followed by a second wave of signals that lead to the stabilisation of the platelet aggregate.

The first step in the recruitment of platelets to the injured vessel wall is mediated by the platelet glycoprotein (Gp) Ib/IX/V receptor complex, one of the most abundant receptors present at the platelet surface. The main ligand for this GpIb/IX/V complex is von Willebrand factor (VWF), a multimeric plasma protein. VWF is able to act as a molecular bridge between components of the exposed subendothelial matrix (in particular collagen) and the platelet GpIb/IX/V complex (Ruggeri ZM (2001) Thromb Res 120-suppl 1:S5). The interaction between VWF and GpIb/IX/V is of particular importance under conditions of rapid blood flow as present within arterial and/or stenosed vessels. Indeed, studies using VWF-deficient mice have shown that the absence of VWF is associated with delayed platelet adhesion and defective thrombus formation (Denis CV and Wagner DD (2007) ATVB 27:728). Moreover, thrombus formation does not lead to occlusive thrombi in the absence of VWF. Although more prominent in arterial vessels, decreased platelet adhesion, smaller thrombi and absent occlusion are also observed in venous vessels (Chauhan CK et al. (2007) Blood 109:2424).

Interaction between platelets and the subendothelial collagen structures also occurs in a direct manner. So far, two collagen receptors have been identified: the integrin α2β1 and a member of the immunoglobulin superfamily, glycoprotein VI (GpVI). The precise roles of both receptors in the platelet-collagen interaction is still a matter of debate (Nieswandt B and Watson SP (2003) Blood 102:449). At lower shear rates, both receptors have the potential to contribute to the adhesion of platelets to collagen. Under conditions of high blood shear (> 1500 s⁻¹) the contribution of both receptors to initial platelet adhesion is negligible and adhesion is fully dependent on VWF. This does not mean, however, that both receptors are of minor importance. In contrast, both α2β1 and GpVI have been shown to be required for stable adhesion of platelets to a collagen surface (Nieswandt B and Watson SP (2003) Blood 102:449). In addition, both receptors are coupled to intracellular signalling pathways that contribute to further platelet activation and aggregation as well as the stabilisation of platelet aggregates.

Under physiological conditions, thrombus formation is under strict control in order to prevent undesired occlusion of the vasculature. However, several pathological conditions are associated with damage of the vessel wall and subsequent occlusion of the vascular system. For instance, surgical procedures like angioplasty, atherectomy and arterial stenting may lead to formation of occlusive thrombi, restenosis, intimal hyperplasia, myocardial infarction and/or stroke. Treatment dedicated to prevent or reduce athero-thrombotic complications includes the use of thrombolytic, anti-coagulant and/or anti-platelet agents. As for anti-platelet agents, these include inhibitors of ADP-receptors (such as Clopidogrel), thromboxane-formation (such as aspirin) and/or agents that block the fibrinogen receptor GpIIbIIIa (such as Abxicimab). However, these treatments involve a risk of adverse bleeding events since coagulation is generally counteracted throughout the body. Moreover, a potential disadvantage of agents directed to platelet-receptors (such as GpIb/IX/V, α2β1 and GpVI) is that this may lead to (transient) thrombocytopenic side effects. It would therefore be desirable to use an agent which is capable of more specifically counteracting thrombus formation at a site of vessel damage.

The invention provides the insight that a leukocyte associated immunoglobulin-like receptor (LAIR), or a functional equivalent thereof, is capable of counteracting platelet-collagen interaction. Hence, LAIR or a functional equivalent thereof is particularly suitable for counteracting thrombus formation at a site where a blood vessel is damaged and where collagen is exposed to the blood stream. Counteracting platelet-collagen interaction results in less thrombus formation.

Different LAIR molecules are known. LAIR-1 is a unique member of the group of inhibitory receptors because of its broad expression in the immune system. LAIR-1 is a member of the Immunoglobin superfamily (IgSF) and is expressed on the majority of peripheral blood mononuclear cells, including natural killer (NK) cells, T-cells, B-cells, monocytes and Dendritic Cells (DCs), as well as the majority of thymocytes. LAIR-1 is an inhibitory immune receptor that contains two ITIM motifs in its cytoplasmatic tail. Several years ago the inventors identified a second LAIR molecule, LAIR-2, in humans. LAIR-2 is 84% homologous to LAIR-1. LAIR-2 differs from LAIR-1 in that it lacks a transmembrane and cytoplasmic domain, which indicates a soluble character. Recently, the present inventors have shown that LAIR is capable of specifically binding collagen (PCT/NL2007/050093). A use of LAIR, or a functional equivalent thereof, for counteracting platelet-collagen interaction has not been disclosed before the present invention. Since multiple binding sites for human LAIR-1 on both human collagens I and III are present, and since platelet binding to collagen involves several independent regions (sequences recognizing VWF, α2β1 or GpVI), it was not known before the present invention whether LAIR and platelets are capable of binding collagen simultaneously, or whether collagen-LAIR interaction interferes with platelet-collagen interaction.

A functional equivalent of a LAIR is defined herein as a molecule which has 70% or more, but less than 100%, sequence identity with LAIR and which has at least one same property as LAIR in common irrespective of quantitative considerations. Said functional equivalent is capable of counteracting platelet-collagen interaction, albeit not necessarily to the same extent as LAIR. Such functional equivalent for instance comprises:
- a polypeptide wherein one or more amino acid residues are added to the native sequence of LAIR;
- a LAIR molecule wherein from about one to about 150 amino acid residues are deleted, and optionally substituted by one or more amino acid residues; and/or
- a LAIR derivative wherein at least one amino acid residue has been covalently modified so that the resulting product has a non-naturally occurring amino acid. Preferably, a functional equivalent of LAIR has an amino acid sequence having at least about 75% amino acid sequence identity with LAIR, preferably at least about 80%, more preferably at least about 85%, more preferably at least about 90%, most preferably at least about 95%. The higher the sequence identity, the more closely said functional equivalent resembles LAIR.

A preferred example of a functional equivalent of LAIR-1 is a LAIR-1 molecule lacking its transmembrane domain and its cytoplasmic tail. The resulting molecule, which comprises the LAIR-1 ectodomain, is a secreted LAIR molecule. It is of course possible to connect two - or more - LAIR-1 ectodomains in order to form a dimer - or multimer. Such dimers and multimers are also functional equivalents of LAIR-1.

The term "functional equivalent of LAIR" also encompasses a LAIR molecule which has been altered such that its capability of counteracting platelet-collagen interaction remains essentially the same in kind, not necessarily in amount. Such functional equivalent is provided in many ways, for instance through conservative amino acid substitution, whereby an amino acid residue is substituted by another residue with generally similar properties (size, hydrophobicity, etc), such that the overall functioning is likely not to be seriously affected. Alternatively, or additionally, a functional equivalent is for instance obtained by screening of a peptide library.

By "counteracting platelet-collagen interaction" is meant herein that a binding event between a collagen and a platelet is at least in part inhibited or delayed, and/or that a platelet which is already bound to collagen is more quickly released and/or more often released. As a result, less thrombus formation occurs within a given time frame.

Collagens are the most abundant proteins in vertebrates. Collagens are known to play crucial roles in the development, morphogenesis, and growth of many tissues. Besides their mechanical properties, collagens serve as substrates for cell attachment, migration, coagulation and mediate signalling events by binding to several cell surface receptors such as integrins, discoidin domain receptors (DDRs), glycoprotein VI, and proteoglycan receptors. The subendothelial matrix of a vessel wall comprises significant amounts of collagen. Hence, when a vessel wall is injured, collagen is exposed to the blood stream.

According to the present invention, LAIR or a functional equivalent thereof is particularly suitable for counteracting platelet-collagen interaction. As shown in the examples, administration of LAIR to blood which is exposed to collagen results in a strong inhibition of adhesion and aggregation of platelets at the collagen surface. One aspect of the present invention therefore provides a use of a leukocyte associated immunoglobulin-like receptor (LAIR), or a functional equivalent thereof, for the preparation of a medicament for counteracting platelet-collagen interaction.

In one embodiment LAIR-1, or a functional equivalent thereof, is used. Alternatively, or additionally, LAIR-2 or a functional equivalent thereof is used.

In a preferred embodiment a secreted LAIR, or a functional equivalent thereof, is used for the preparation of a medicament for counteracting platelet-collagen interaction. A secreted LAIR is a LAIR molecule which is normally secreted within an animal's body. This does not mean that such secreted LAIR is never bound to a cell. As long as at least 10%, preferably at least 25%, more preferably at least 40% of a particular LAIR variant is secreted after production by cells of an animal, said LAIR variant is called a secreted LAIR. As used herein, the term "animal" encompasses non-human animals as well as human individuals. A use of a secreted LAIR as a medicament has the advantage that said secreted LAIR is easy to dose and that it is administered in a cell-free environment. This is more convenient as compared to LAIR-1, which is for instance administered in a cell-bound form or as a liposome-vesicle. A secreted LAIR as used in the invention is preferably LAIR-2.

In a further preferred embodiment a soluble LAIR, or a functional equivalent thereof, is used for the preparation of a medicament for counteracting platelet-collagen interaction. A soluble LAIR is defined herein as a LAIR molecule that is at least 80% soluble in an aqueous environment, such as for instance blood. A LAIR molecule is at least 80% soluble in an aqueous environment when at least 80% of the LAIR molecules of a given LAIR aliquot dissolves in said aqueous environment. A soluble LAIR comprises any soluble LAIR component that is capable of counteracting platelet-collagen interaction. A soluble LAIR for instance comprises a recombinant LAIR, preferably a recombinant LAIR-1. Alternatively, or additionally, a recombinant LAIR-2 is used. Use of a soluble LAIR is preferred because the bioavailability of water-soluble compounds is generally better as compared to non water-soluble compounds.

A further embodiment provides a method for at least in part preventing, counteracting and/or inhibiting platelet-collagen interaction, comprising providing LAIR, or a functional equivalent thereof, to an environment comprising collagen and platelets. Said environment preferably comprises the blood circulation of an animal. In a preferred embodiment, a therapeutically effective amount of LAIR, or a functional equivalent thereof, is administered to a human individual in order to at least in part counteract and/or inhibit platelet-collagen interaction within said individual. This method is for instance suitable for at least in part preventing and/or delaying thrombus formation in an injured blood vessel of said human individual. In one embodiment said LAIR comprises LAIR-1. Alternatively, or additionally, said LAIR comprises LAIR-2. Most preferably, a secreted and/or soluble LAIR is used.

In one embodiment of the invention, use is made of LAIR which is endogenously present in an animal, for instance a human individual. Enhancing the amount and/or activity of endogenous LAIR in an individual results in at least partial inhibition of platelet-collagen interaction within said individual. Means for enhancing the amount and/or activity of endogenous LAIR are known in the art. The amount of LAIR is for instance increased by administration of a compound capable of enhancing LAIR expression. Alternatively, or additionally, the amount of LAIR is increased by decreasing breakdown of LAIR in an animal's body. Suitable compounds capable of decreasing breakdown of LAIR for instance include blockers of clearance receptors that remove LAIR from the circulation, and/or compounds that reduce renal clearance of LAIR. Breakdown of LAIR is for instance obtained via chemical modification using pegylation (the coupling of polyethylene glycol components to amino acid or carbohydrate residues present within a LAIR molecule), and/or poly-sialylation (the coupling of polysialyl residues to amino acid or carbohydrate residues present within a LAIR molecule). LAIR expression is for instance increased by stimulation of LAIR promoter activity (in particular relevant for LAIR-2) and/or by increasing molecular shedding of LAIR-1 via for instance metalloproteases that proteolytically separate (part of) the LAIR-1 ectodomain from the transmembrane and intracellular region. Further provided is therefore a use of a compound capable of enhancing the amount and/or activity of LAIR in an individual for the preparation of a medicament for counteracting platelet-collagen interaction within said individual. In a preferred embodiment, use is made of a compound capable of enhancing the amount and/or activity of LAIR-2 in an individual for the preparation of a medicament for counteracting platelet-collagen interaction within said individual, since LAIR-2 is a secreted, soluble LAIR which is naturally present within the circulation. Such compound is particularly suitable for preventing, counteracting and/or treating a condition related to thrombus formation, athero-thrombosis, restenosis and/or intimal hyperplasia. One embodiment therefore provides a method for preventing, counteracting and/or treating a condition related to thrombus formation, athero-thrombosis, restenosis and/or intimal hyperplasia, comprising administering to a subject in need thereof a therapeutically amount of a compound capable of enhancing the amount and/or activity of endogenous LAIR-2 within said subject.

A medicament according to the invention is preferably used for counteracting adhesion of platelets to collagen, so that the initial step of thrombus formation is at least in part inhibited and/or delayed. One embodiment therefore provides a use according to the invention for the preparation of a medicament for counteracting adhesion of platelets to collagen. In one embodiment, activation of platelets is counteracted so that platelet-platelet interaction, needed for thrombus growth, is at least in part inhibited and/or delayed. A use of a LAIR, or a functional equivalent thereof, for the preparation of a medicament for counteracting activation of platelets is therefore also provided.

A medicament according to the invention is suitable for at least in part treating, counteracting and/or preventing a condition related to thrombus formation. Such condition for instance comprises an athero-thrombotic complication such as thrombosis, restenosis, intimal hyperplasia, myocardial infarction and/or stroke. Thrombosis is a disorder involving the formation of a thrombus that prevents normal blood flow. Thrombi may develop anywhere in the cardiovascular system. Arterial thrombi usually begin at a site of endothelial injury (eg. an atherosclerotic plaque) or turbulence (eg a bifurcation) somewhere within the arterial vasculature. An area of attachment (eg the exposed subendothelial matrix) allows the recruitment of platelets, the starting point of a platelet-rich thrombus. Arterial thrombi are usually occlusive, thereby obstructing blood flow. (Pathological basis of disease 6th edition; page 126; Ed: Cotran RS, Kumar V and Collins T; 1999; W.B. Saunders Company ; Philadelphia PE). When such blood clot seriously hampers blood flow through a vessel, for instance a coronary artery, this results in a myocardial infarction, or stroke. When a coronary artery is (partially) obstructed, the stenosed vessel may be surgically repaired via percutaneous transluminal coronary angioplasty (PTCA). Percutaneous coronary intervention encompasses a variety of procedures used to treat patients with diseased arteries of the heart, for example, chest pain caused by a buildup of fats, and/or other substances from the blood (referred to as plaque) that reduce blood flow to a near trickle, or a heart attack caused by a large blood clot that completely blocks the artery. PTCA (which can eventually be performed in conjugation with stenting) usually leads to initial success, but may be compromised by re-narrowing of the vessel at the site of intervention, *ie*. restenosis. Restenosis is a process involving distinct biological processes such as platelet adhesion and thrombus formation at the injured vessel wall and proliferation and migration of vascular smooth muscle cells leading to neo-intima formation.

Further disorders that are at least in part counteracted and/or prevented with a medicament according to the present invention comprise acute arterial obstruction (eg. unstable angina, myocard infarction, stroke) due to atherosclerosis, diabetes or other disorders within the arterial vessels in which the patient has not received previous treatment; and secondary thrombotic complications for instance due to percutaneous coronary intervention (with or without stenting) to treat an acute arterial obstruction.

Further provided is therefore a use of a LAIR, or a functional equivalent thereof, according to the invention for the preparation of a medicament for at least in part treating, counteracting and/or preventing a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction. A method for preventing, counteracting and/or treating a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction, comprising administering to a subject in need thereof a therapeutically effective amount of LAIR, or a functional equivalent thereof, is also provided. In one embodiment said LAIR comprises LAIR-1. Alternatively, or additionally, said LAIR comprises LAIR-2. Most preferably, a secreted and/or soluble LAIR is used.

Plaques are often removed from a blood vessel using atherectomy, angioplasty and/or arterial stenting. During atherectomy, plaque is removed from the walls of blood vessels using instruments inserted via a catheter. Angioplasty is a surgical procedure wherein a catheter is inserted into a narrowed blood vessel. This procedure is conventionally carried out with a balloon catheter. Inflation of the balloon results in compression of the plaque and enlarging of the inner diameter of the blood vessel. Instead of a conventional balloon catheter, stents are mostly used nowadays. A stent is a small, flexible tube made of medical-grade plastic or metal. An implanted stent serves as a scaffold that keeps the blood vessel open. However, vessel unblocking procedures may damage blood vessels to some extent, which may lead to formation of occlusive thrombi, restenosis and intimal hyperpasia. Therefore, according to the present invention, a device is preferably used which comprises LAIR or a functional equivalent thereof, so that platelet-collagen interaction at a site of blood vessel injury is locally counteracted. The close proximity of LAIR, or a functional equivalent thereof, to a damaged blood vessel site is particularly advantageous for counteracting thrombus formation.

A preferred embodiment therefore provides a device for use in blood vessel unblocking techniques such as for instance angioplasty, atherectomy and/or arterial stenting, said device comprising LAIR, or a functional equivalent thereof. Said device preferably comprises a stent, because stents are widely used for keeping blood vessels open. In one embodiment said device comprises a slow-release formulation comprising LAIR, or a functional equivalent thereof. A slow-release formulation provides the advantage that the local concentration of LAIR at a site of a blood vessel which is possibly injured and/or prone to thrombus formation remains elevated during a prolonged period of time. Said device preferably comprises a secreted and/or soluble LAIR, or a functional equivalent thereof. One preferred embodiment provides a device according to the invention, wherein said LAIR comprises LAIR-1. Alternatively, or additionally, a device according to the invention preferably comprises LAIR-2.

Further provided is a pharmaceutically composition comprising LAIR, or a functional equivalent thereof, and a pharmaceutically suitable carrier, diluent or excipient. Such pharmaceutical composition is capable of counteracting platelet-collagen interaction. Therefore, it is particularly suitable for at least in part counteracting, treating or preventing a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction. A pharmaceutical composition according to the invention preferably comprises LAIR-1. Most preferably, a secreted or soluble LAIR-1 variant is used.

In one embodiment, LAIR is used as the sole pharmaceutically active agent for counteracting platelet-collagen interaction. In an alternative embodiment, however, LAIR is used in combination with at least one other anti-thrombotic agent. Such combination is suitable for increasing pharmaceutical activity. One embodiment therefore provides a kit of parts comprising an anti-thrombotic agent and LAIR, or a functional equivalent thereof. Anti-thrombotic agents other than LAIR are well known in the art. Non-limiting examples comprise thrombolytic, anti-coagulant and/or anti-platelet agents, such as inhibitors of ADP-receptors (for instance Clopidogrel), thromboxane formation (for instance aspirin) and/or agents that block the GpIIbIIIa receptor (for instance Abxicimab), and/or chemically synthesized inhibitors like for instance Eptifibatide (Integrilin) or Tirofiban (Aggrastat). Said LAIR preferably comprises LAIR-1 and/or LAIR-2, or a functional equivalent thereof. A kit of part according to the invention most preferably comprises a secreted and/or soluble LAIR, or a functional equivalent thereof.

A use of LAIR, or a functional equivalent thereof, for counteracting platelet-collagen interaction is also provided. Besides *in vivo* applications, such as for instance those described above, LAIR, or a functional equivalent thereof, is also suitable for use *in vitro*, for instance for testing candidate compounds for their capability of counteracting platelet-collagen interaction. Such candidate compounds, which will be suitable for use as an anti-thrombotic agent, will compete with LAIR. In another application, LAIR, or a functional equivalent thereof, is used in order to test candidate compounds *in vitro* for their capability of enhancing platelet-collagen interaction. Such candidate compounds, which will be suitable for use as an anti-bleeding agent, will counteract the anti-thrombotic activity of LAIR.

Now that the invention provides the insight that LAIR, or a functional equivalent thereof, is capable of counteracting platelet-collagen interaction, it has become possible to interfere with platelet-collagen interaction in various ways. Administration of LAIR, or a functional equivalent thereof, to an environment comprising collagen and platelets, such as for instance an injured blood vessel, results in at least partial inhibition of thrombus formation. It is, however, also possible to use a compound which is capable of specifically interacting in the binding of LAIR and collagen. For instance, a compound capable of counteracting LAIR-collagen interaction is capable of enhancing platelet-collagen interaction. Such compound for instance comprises a LAIR-specific antibody, or a functional fragment of said antibody, or a peptide derived from collagen. Furthermore, a compound capable of enhancing LAIR-collagen interaction is capable of counteracting platelet-collagen interaction. Such compound is for instance capable of improving the stability of LAIR and/or the stability of a LAIR-collagen complex. One aspect of the invention therefore provides a method for interfering in platelet-collagen interaction, comprising providing a compound capable of specifically interacting in the binding of LAIR and collagen to an environment comprising collagen and platelets. Said environment preferably comprises the blood circulation of an animal. In a preferred embodiment, said compound is administered to a human individual in order to influence platelet-collagen interaction. For instance, a compound capable of enhancing LAIR-collagen interaction - thereby counteracting platelet-collagen interaction - is used for at least in part preventing and/or delaying thrombus formation in an injured blood vessel of said human individual. Alternatively, a compound capable of counteracting LAIR-collagen interaction - thereby enhancing platelet-collagen interaction - is used for at least in part counteracting a bleeding disorder.

It is clear from the above that a compound capable of specifically interacting in the binding of LAIR and collagen is particularly suitable for the preparation of a medicament. One embodiment therefore provides a use of a compound capable of enhancing the binding of LAIR to collagen for the preparation of a medicament for counteracting platelet-collagen interaction. Preferably, said medicament is for at least in part preventing, counteracting and/or treating a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction. A method for at least in part preventing, counteracting and/or treating a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction, the method comprising administering to a subject in need thereof a therapeutically effective amount of a compound capable of enhancing the binding of LAIR to collagen, is also herewith provided.

A further embodiment provides a use of a compound capable of counteracting the binding of LAIR to collagen for the preparation of a medicament for enhancing platelet-collagen interaction. Preferably, said medicament is for at least in part treating, counteracting and/or preventing a bleeding disorder. A method for at least in part preventing, counteracting and/or treating a bleeding disorder, comprising administering to a subject in need thereof a therapeutically effective amount of a compound capable of counteracting the binding of LAIR to collagen, is therefore also provided.

In one embodiment a compound is used which is capable of specifically enhancing, or counteracting, the binding of LAIR-1 to collagen. Preferably, however, a compound is used which is capable of specifically enhancing, or counteracting, the binding of LAIR-2 to collagen since LAIR-2 is secreted within a human body and therefore endogenously present in the circulation of a human individual.

In one embodiment, a compound capable of specifically interacting in the binding of LAIR and collagen is used as the sole active agent for influencing platelet-collagen interaction. In an alternative embodiment, however, a compound capable of specifically interacting in the binding of LAIR and collagen is used in combination with at least one other anti-thrombotic or anti bleeding agent. Such combination is suitable for increasing pharmaceutical activity. One embodiment therefore provides a kit of parts comprising a compound capable of specifically enhancing the binding of LAIR to collagen, and an anti-thrombotic agent. Anti-thrombotic agents are well known in the art, as described herein above.

Another embodiment provides a kit of parts comprising a compound capable of specifically counteracting the binding of LAIR to collagen, and an anti bleeding agent. Anti bleeding agents are also well known in the art. A non-limiting example comprises activated factor VII (Novoseven).

A use of a compound capable of specifically interacting in the binding of LAIR and collagen for influencing platelet-collagen interaction is also provided. Besides *in vivo* applications, such as for instance those described above, a compound capable of specifically interacting in the binding of LAIR and collagen is also suitable for use *in vitro*, for instance for testing candidate compounds for their capability of influencing (enhancing or counteracting) platelet-collagen interaction. A use of a compound capable of specifically counteracting the binding of LAIR and collagen for enhancing platelet-collagen interaction is therefore also provided, as well as a use of a compound capable of specifically enhancing the binding of LAIR and collagen, for counteracting platelet-collagen interaction.

In one embodiment, a device for use in blood vessel unblocking techniques such as for instance angioplasty, atherectomy and/or arterial stenting is provided with a compound capable of specifically enhancing the binding of LAIR and collagen, so that platelet-collagen interaction at a site of blood vessel injury is locally counteracted. The close proximity of such compound to a damaged blood vessel site is particularly advantageous for counteracting thrombus formation. Said device preferably comprises a stent, because stents are widely used for keeping blood vessels open. In one embodiment said device comprises a slow-release formulation comprising said compound capable of specifically enhancing the binding of LAIR and collagen.

The invention is further explained in the following examples. These examples do not limit the scope of the invention, but merely serve to clarify the invention.

### Examples

### Example 1

### Soluble LAIR molecules variants as potential antithrombotic agents

The adhesion of platelets to collagen is a prerequisite to induce platelet activation and aggregation. It is known that collagen-receptors GpVI and α2β1 are both of importance for this process. Platelet may interact with collagen in an indirect manner, in which von Willebrand factor acts as a molecular bridge between collagen and platelet-receptor GpIb/IX/V complex. Indeed, inhibitors directed to the prevention of platelet-collagen interactions are currently being developed as anti-thrombotic agents.

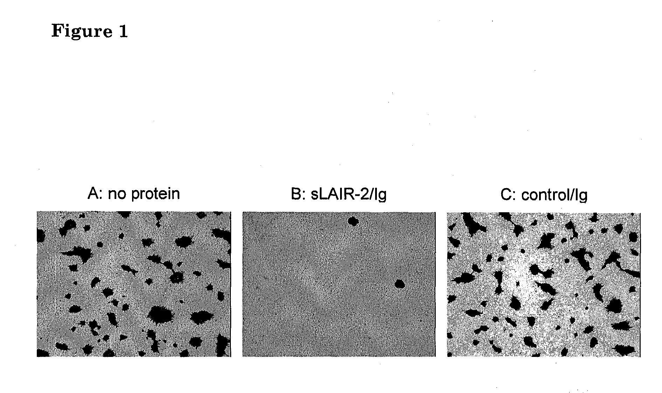

We considered the possibility that soluble LAIR-variants (LAIR-2 and/or recombinant derivatives of LAIR-1) may be used to interfere with platelet-collagen interactions. This was tested in experiments in which thrombus-formation under flow was initiated by the perfusion of blood over a collagen-surface in the presence or absence of soluble LAIR-2/Ig fusion protein, LAIR-1/Ig fusion protein, or a control protein. Whereas normal thrombus-formation was observed under control conditions, the adhesion and aggregation of platelets at the collagen-surface was completely inhibited in the presence of recombinant LAIR-2/Ig or LAIR-1/Ig (Fig. 1, 2 and 3). Thus, LAIR has a high anti-thrombotic potential.

### Methods

Human placenta type III collagen (Sigma) was immobilized onto Thermanox^{®} coverslips by a coating procedure resulting in a concentration of 30 µg/cm². Coverslips were blocked (1 hr, 20°C) with 1% human albumin in phosphate-buffered saline (PBS). Perfusions were carried out with citrated human blood (0.34% Na-citrate f.c.) in a triplicate single-passage parallel-plate perfusion chamber as described (Sixma et al, Thromb Res 1998; 92:S43-S46). Coverslips were preincubated with LAIR-2 in PBS and with PBS alone as a control. In a second series of experiments, LAIR-2 in PBS was added to blood and again PBS served as control. The blood was perfused over the collagen-coated coverslips for 5 min at a shear rate of 300 s⁻¹. After perfusion, slides were washed with Hepes buffer (10 mmol/L Hepes, 150 mmol/L NaCl, pH 7.35) and fixed in 0.5% glutaraldehyde in PBS. Slides were dehydrated in methanol and stained with May-Grünwald and Giemsa (Sakariassen et al, J.Lab.Clin.Med. 1983; 102:522-35). Platelet adhesion was evaluated using computer-assisted analysis with OPTIMAS 6.0 software [Dutch Vision Systems (DVS), Breda, the Netherlands]. Platelet attachment to surface-bound fibrinogen was expressed as surface coverage (expressed as percentage).

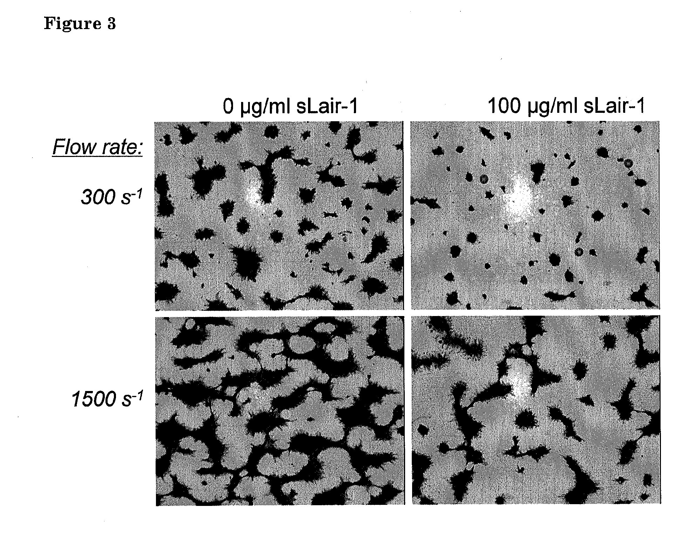

In a third experiment, collagen type III-coated coverslips were perfused with whole blood in the absence or presence of soluble LAIR-1/Ig fusion protein. The results are shown in Figure 3. The presence of LAIR-1/Ig resulted in 49 % and 47 % inhibition of platelet adhesion at a shear rate of 300 s⁻¹ (upper panels) or 1500 s⁻¹ (lower panels) respectively.

### Example 2

### Soluble LAIR blocks binding of von Willebrand factor to collagen I and III

Von Willebrand factor (VWF) is well known for its ability to interact with various types of collagen, including collagen I and collagen III. Binding of VWF to collagen is believed to be associated with a conformational change, which allows VWF to interact with platelet receptor GpIb/IX/V. As such, VWF bridges the collagen surface and platelets, a process that is particularly relevant under conditions of rapid flowing blood.

Given the high number of LAIR-molecules that can bind to collagen (10 mol per mol collagen), we considered the possibility that soluble LAIR molecules can interfere with the binding of VWF to collagen.

### Methods

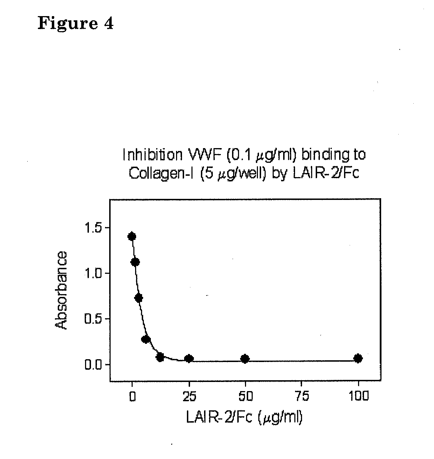

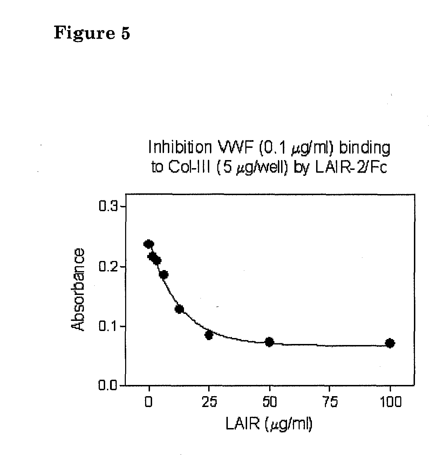

Microtiter wells were coated with collagen I or collagen III (50 µg/ml) in phosphate-buffered saline (PBS) (pH 7.4) for 16 h at 4°C. Then wells were washed three times with PBS/0.01 % Tween-20. Purified plasma-derived VWF was diluted to a concentration of 0.4 µg/ml. Serial dilutions of recombinant LAIR-2/Fc were prepared to obtain concentrations ranging between 0 and 133.3 µg/ml. We then combined 25 µl of VWF solution and 75 µl of LAIR-2/Fc solution, resulting in 100 µl containing 0.1 µg/ml VWF and 0-100 µg/ml LAIR2-Fc. All dilutions were made in PBS/0.01 % Tween-20 supplemented with 2 mg/ml bovine serum albumin. The VWF * LAIR-2/Fc solution was incubated with immobilized collagen I or III for 1.5 h at 37°C. After washing three times with PBS/0.01 % Tween-20, wells were incubated with horseradish-conjugated polyclonal anti-human VWF antibodies (DAKO, Denmark), 1:3000 diluted in PBS/0.01 % Tween-20 for 1.5 h at 37°C. After three washes with PBS/0.01 % Tween-20, bound antibodies detecting residual VWF were visualized via incubation with 3-3'-5-5'-tetramethylbenzidine. Figure 4 shows the effect of LAIR-2/Fc on binding of VWF to collagen I. VWF binding is completely inhibited in a dose-dependent manner, in which half-maximal inhibition is observed at a concentration of 2.9 µg/ml LAIR-2/Fc. Figure 5 shows the effect of LAIR-2/Fc on binding of VWF to collagen III. VWF binding is completely inhibited in a dose-dependent manner, in which half-maximal inhibition is observed at a concentration of 8.9 µg/ml LAIR-2/Fc.

### Brief description of the drawings

**Figure 1****: Soluble LAIR-2/Ig fusion protein blocks platelet adhesion to collagen.** Collagen type III-coated coverslips were perfused with whole blood in the absence (A) or presence of soluble LAIR-2/Ig fusion protein (B) or a control Ig-fusion protein (C). The presence of LAIR-2/Ig resulted in amore than 95 % inhibition of platelet adhesion.

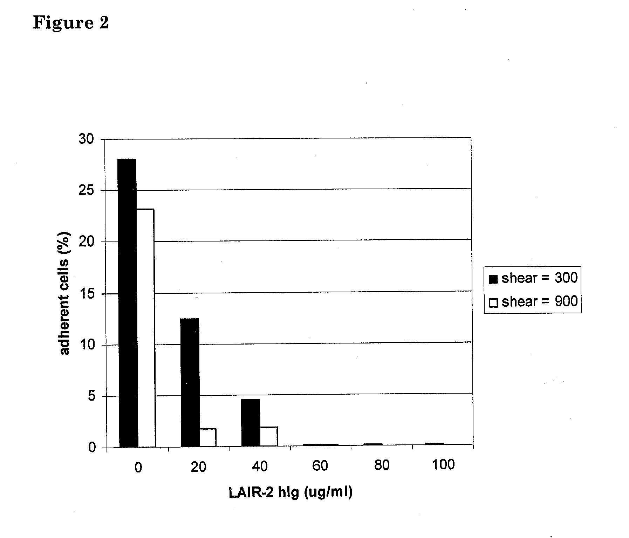

**Figure 2****: Soluble LAIR-2/Ig fusion protein blocks platelet adhesion to collagen at high and low shear rate.** Collagen type III-coated coverslips were perfused with whole blood in the presence increasing concentrations of soluble LAIR-2/Ig fusion protein. Perfusion was performed at a shear rate of 300 s⁻¹ (black bars) or 900 s⁻¹ (open bars).
**Figure 3****: Soluble LAIR-1/Ig fusion protein blocks platelet adhesion to collagen.** Collagen type III-coated coverslips were perfused with whole blood in the absence (left panels) or presence (right panels) of soluble LAIR-1/Ig fusion protein. The presence of LAIR-1/Ig resulted in 49 % and 47 % inhibition of platelet adhesion at a shear rate of 300 s⁻¹ (upper panels) or 1500 s⁻¹ (lower panels) respectively.
**Figure 4****: Inhibition of VWF binding to collagen-I by LAIR-2/Fc.**
**Figure 5****: Inhibition of VWF binding to collagen-III by LAIR-2/Fc.**

### References

Barnes CS, Krafft B, Frech M, Hofmann UR, Papendieck A, Dahlems U, Gellissen G & Hoylaerts MF (2001) Production and characterization of saratin, an inhibitor of von Willebrand factor-dependent platelet adhesion to collagen. Semin Thromb Hemost 27:337-348.
Chauhan CK et al. (2007) Blood 109:2424
Connolly TM, Jacobs JW & Condra C (1992) An inhibitor of collagen-stimulated platelet activation from the salivary glands of the Haementeria officinalis leech. I. Identification, isolation, and characterization. J Biol Chem 267:6893-6898.
Denis CV and Wagner DD (2007) Platelet adhesion receptors and their ligands in mouse models of thrombosis. Arterioscler Thromb Vasc Biol 27:728-739
Keller PM, Schultz LD, Condra C, Karczewski J & Connolly TM (1992) An inhibitor of collagen-stimulated platelet activation from the salivary glands of the Haementeria officinalis leech. II. Cloning of the cDNA and expression. J Biol Chem 267:6899-6904.
Lasser G, Guchhait P, Ellsworth JL, Sheppard P, Lewis K, Bishop P, Cruz MA, Lopez JA, Fruebis J (2006) C1qTNF-related protein-1 (CTRP-1): a vascular wall protein that inhibits collagen-induced platelet aggregation by blocking VWF binding to collagen. Blood 107:423-430
Munro R, Jones CP & Sawyer RT (1991) Calin - a platelet adhesion inhibitor from the saliva of the medicinal leech. Blood Coagul Fibrinolysis 2:179-184.
Nieswandt B and Watson SP (2003) Platelet-collagen interaction: is GpVI the central receptor. Blood 102:449-461
Pinto Slottow TL and Waksman R (2007) Overview of the 2006 Food and Drug Administration Circulatory System Devices. Panel meeting on drug-eluting stent thrombosis. Catheter Cardiovasc Interv 69:1064-74.
Ruggeri ZM (2001) Thromb Res 120-suppl 1:S5
Sixma JJ, de Groot PG, van Zanten H, Ijsseldijk M. A new perfusion chamber to detect platelet adhesion using a small volume of blood. Thromb Res 1998;92:S43-S46.
Sakariassen KS, Aarts PA, de Groot PG, Houdijk WP, Sixma JJ. A perfusion chamber developed to investigate platelet interaction in flowing blood with human vessel wall cells, their extracellular matrix, and purified components. J Lab Clin Med 1983;102:522-35.
White TC, Berny MA, Robinson DK, Yin H, DeGrado WF, Hanson SR & McCarty OJ (2007) The leech product saratin is a potent inhibitor of platelet integrin α2β1 and von Willebrand factor binding to collagen. FEBS Journal 274:1481-1491.
Pathological basis of disease 6th edition; page 126; Ed: Cotran RS, Kumar V and Collins T; 1999; W.B. Saunders Company ; Philadelphia PE

## Claims

1. Use of a leukocyte associated immunoglobulin-like receptor (LAIR), or a functional equivalent thereof, for the preparation of a medicament for counteracting platelet-collagen interaction.

2. Use according to claim 1, wherein said LAIR or functional equivalent comprises a secreted and/or soluble LAIR or a functional equivalent thereof.

3. Use according to claim 1 or 2, wherein said LAIR comprises LAIR-1.

4. Use according to claim 1 or 2, wherein said LAIR comprises LAIR-2.

5. Use of a compound capable of enhancing the amount and/or activity of LAIR-2 in an individual for the preparation of a medicament for counteracting platelet-collagen interaction within said individual.

6. Use according to any one of claims 1-5, for the preparation of a medicament for counteracting adhesion of platelets to collagen.

7. Use according to any one of claims 1-6, for the preparation of a medicament for counteracting activation of platelets.

8. Use according to any one of claims 1-7, for the preparation of a medicament for at least in part treating, counteracting and/or preventing a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction.

9. A device for use in angioplasty, atherectomy and/or arterial stenting comprising LAIR, or a functional equivalent thereof.

10. A device according to claim 9, wherein said device comprises a stent.

11. A device according to claim 9 or 10, wherein said device comprises a slow-release formulation comprising LAIR, or a functional equivalent thereof.

12. A device according to any one of claims 9-11, wherein said LAIR or functional equivalent comprises a secreted and/or soluble LAIR or functional equivalent thereof.

13. A device according to any one of claims 9-12, wherein said LAIR comprises LAIR-1.

14. A device according to any one of claims 9-12, wherein said LAIR comprises LAIR-2.

15. A method for at least in part preventing, counteracting and/or inhibiting platelet-collagen interaction, comprising providing LAIR, or a functional equivalent thereof, to an environment comprising collagen and platelets.

16. A method for at least in part preventing, counteracting and/or treating a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction, the method comprising administering to a subject in need thereof a therapeutically effective amount of LAIR, or a functional equivalent thereof.

17. A method according to claim 15 or 16, wherein said LAIR or functional equivalent comprises a secreted and/or soluble LAIR or functional equivalent thereof.

18. Method according to any one of claims 15-17, wherein said LAIR comprises LAIR-1.

19. Method according to any one of claims 15-17, wherein said LAIR comprises LAIR-2.

20. A method for at least in part preventing, counteracting and/or treating a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction, the method comprising administering to a subject in need thereof a therapeutically amount of a compound capable of enhancing the amount and/or activity of endogenous LAIR-2 within said subject.

21. A pharmaceutically composition comprising LAIR-1, or a functional equivalent thereof, and a pharmaceutically suitable carrier, diluent or excipient.

22. A kit of parts comprising:
- an antithrombotic agent; and
- LAIR or a functional equivalent thereof.

23. Use of LAIR, or a functional equivalent thereof, for counteracting platelet-collagen interaction.

24. A method for interfering in platelet-collagen interaction, comprising providing a compound capable of specifically interacting in the binding of LAIR and collagen to an environment comprising collagen and platelets.

25. Use of a compound capable of enhancing the binding of LAIR to collagen for the preparation of a medicament for counteracting platelet-collagen interaction.

26. Use of a compound capable of counteracting the binding of LAIR to collagen for the preparation of a medicament for enhancing platelet-collagen interaction.

27. Use according to claim 25 or 26, wherein said LAIR comprises LAIR-1.

28. Use according to claim 25 or 26, wherein said LAIR comprises LAIR-2.

29. Use according to claim 25 or 27-28, wherein said medicament is for at least in part preventing, counteracting and/or treating a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction.

30. Use according to any one of claims 26-28, wherein said medicament is for at least in part treating, counteracting and/or preventing a bleeding disorder.

31. A method for at least in part preventing, counteracting and/or treating a condition related to thrombus formation, athero-thrombosis, restenosis, intimal hyperplasia, myocardial infarction, stroke, acute arterial obstruction (eg. unstable angina) due to atherosclerosis, diabetes or other disorders within the arterial vessels, and/or secondary thrombotic complications, for instance due to percutaneous coronary intervention (with or without stenting) to treat an acute arterial obstruction, the method comprising administering to a subject in need thereof a therapeutically effective amount of a compound capable of enhancing the binding of LAIR to collagen.

32. A method for at least in part preventing, counteracting and/or treating a bleeding disorder, comprising administering to a subject in need thereof a therapeutically effective amount of a compound capable of counteracting the binding of LAIR to collagen.

33. A kit of parts comprising:
- an anti bleeding agent; and
- a compound capable of specifically counteracting the binding of LAIR to collagen.

34. Use of a compound capable of specifically counteracting the binding of LAIR and collagen, for enhancing platelet-collagen interaction.

35. Use of a compound capable of specifically enhancing the binding of LAIR and collagen, for counteracting platelet-collagen interaction.
